Europäisches Patentamt

European Patent Office

Office européen des brevets

(11) Publication number: **0 381 653**
**A2**

## (12) EUROPEAN PATENT APPLICATION

(21) Application number: **90850037.4**

(22) Date of filing: **26.01.90**

(51) Int. Cl.⁵: **G01N 29/00**

(30) Priority: **01.02.89 SE 8900337**

(43) Date of publication of application:
**08.08.90 Bulletin 90/32**

(84) Designated Contracting States:
**AT BE CH DE FR GB IT LI NL SE**

(71) Applicant: **AB BOFORS**

**S-691 80 Bofors(SE)**

(72) Inventor: **Yström, Lars**
**V Nobelvägen 1**
**S-691 33 Karlskoga(SE)**
Inventor: **Jonsson, Sune**
**V. Rävasgatan 2**
**S-691 35 Karlskoga(SE)**

(74) Representative: **Olsson, Gunnar**
**Nobel Corporate Services Patents and**
**Trademarks**
**S-691 84 Karlskoga(SE)**

(54) **Method and device for determining the degree of adherence between an explosive and the delimiting surface of a projectile.**

(57) Ultrasound is used to determine the degree of adherence between an explosive (4) and a delimiting surface of a delimiting wall (2, 3). The measurements are carried out in two different runs, on the one hand in the form of a preparatory measurement run in which the explosive has not yet been applied against the delimiting surface and, on the other hand, in the form of a main measurement run in which the explosive has been applied. In the measurement run without explosive, the delimiting surface may if appropriate have been provided with a lacquer layer (5) designed to increase the adherence of the explosive. In both measurement runs, two different measurement series are carried out. The ultrasound is directed towards either the inner surface of the delimiting wall or its outer surface. Reflected ultrasonic signals are received and recorded. Ratios between related inner surface echoes and outer surface echoes are calculated, and the difference between the ratios for the preparatory measurement run and the main measurement run represents a very good measurement of the degree of adherence.

Figur 1

EP 0 381 653 A2

# Method and device for determining the degree of adherence between an explosive and the delimiting surface of a projectile

## TECHNICAL FIELD

The present invention relates to a method for determining the degree of adherence between an explosive and a delimiting surface, which can consist for example of the inner surface of a delimiting wall in an ammunition unit, for example in the form of a projectile such as a shell missile or bomb. The method is especially applicable to shell base parts, and the invention also relates to a device for carrying out the method.

## PRIOR ART

The invention is based on the use of an ultrasonic source or another electro-acoustic transducer which is used for emitting ultrasonic signals in the form of pulses. The ultrasonic source can comprise a crystal, for example a piezo-electric crystal which, depending on the electrical alternating voltage applied, is made to oscillate (100 Khz - 50 Mhz) in such a way that the desired ultrasonic signal is obtained. Ultrasonic sources of this type and various areas of application for them are previously known.

In the production of shells, for example, it is often important to be able to determine the degree of adherence between the delimiting wall of the shell and the explosive arranged in the shell. The adherence of the explosive against the inner base of the shell is of particular importance. If this adherence is not satisfactory, an air gap can appear here which, when the shell is fired, can lead to an adiabatic compression with an associated increase in temperature. In some cases this increase in temperature can be so great as to cause an explosion in the bore. As the coefficient of cubical expansion of the explosive is considerably greater than that of the shell material (for example steel), there is an increased risk of gaps developing if the temperature falls. Thus, a shell which appears to be completely free from gaps at room temperature may be found to have considerable gaps at a reduced temperature.

It is previously known to use non-destructive radiographic inspection methods to determine whether large gaps are present, but these methods are unable to detect the small gaps which can be revealed by means of the present invention. Visual examination following destructive sawing-up of shells is an extremely costly method and does not satisfy the requirements set out here. American Patent Specification 4,100,808 describes an assessment of joins by means of ultrasound, but the methodology described there is not applicable to the present invention.

## DESCRIPTION OF THE INVENTION

## TECHNICAL PROBLEM

The aim of the present invention is to provide a method and a device which solve the problem of accurately determining the degree of adherence between an explosive and a delimiting surface. In this respect the invention uses ultrasound generated by an ultrasonic source, which can be of a type which is previously known per se.

## SOLUTION

The feature which principally characterizes the present method is that a series of ultrasonic signals is emitted, from an ultrasonic source situated outside the delimiting wall, in the direction of the join between the explosive and the delimiting surface. Then, from this join, reflected ultrasonic signals or echoes are obtained, whose strength is in relation to the homogeneity of the join. These echoes are received and their amplitudes are recorded. During the same measurement run which has been described above, in which ultrasonic signals are sent towards the join ( = the inner surface of the delimiting wall), a series of ultrasonic signals is also sent towards the opposite surface ( = outer surface) of the delimiting wall. The echoes which are obtained from this latter signal series are also received and recorded.

The two ultrasonic signal series described above, which are both effected in the direction of the delimiting wall with the explosive arranged against the latter, will hereinafter be regarded as relating to the main measurement run. The amplitude values for relevant echoes are designated as follows:

main - inner surface - echo amplitude value (MIE) and

main - outer surface - echo amplitude value (MOE).

Prior to the measurements in the main measurement run, when the explosive has already been arranged against the delimiting wall, corresponding measurements are carried out before the explosive has been applied. These latter measurements without explosive are regarded as relating to the preparatory measurement run.

Thus, in the case of an explosive-filled shell, the preparatory measurement run corresponds to the empty shell and the main measurement run corresponds to the shell filled with explosive.

If a separate lacquer layer is used to improve the adherence between the explosive and the delimiting wall, the preparatory measurement run will be carried out after the lacquer layer has been applied.

The amplitude values which relate to the preparatory measurement run are designated as follows:

preparatory - inner surface - echo amplitude value (PIE) and

preparatory - outer surface - echo amplitude value (POE).

In order to evaluate the measured degree of adherence, the ratios PIE/POE and MIE/MOE are calculated and, thereafter, the difference between these two ratios. This difference represents a very good measurement of the degree of adherence between the explosive and the delimiting surface. In this respect a greater difference indicates better adherence.

A device for carrying out the abovementioned method is principally characterized in that an ultrasonic source is arranged outside the delimiting wall and in that this ultrasonic source can send ultrasonic signal series on the one hand to the inner surface of the delimiting wall and on the other hand to its outer surface. These signal series are to be effected both in the direction of the delimiting wall without applied explosive ( but, if appropriate, with a lacquer layer) (= preparatory measurement run) and in the direction of the delimiting wall with applied explosive (= main measurement run). The device will also include equipment for receiving reflected ultrasonic signals (echoes). This receiving equipment can either consist of the ultrasonic source itself or of another unit. The device will moreover comprise a signal-processing unit which can record the echoes received.

The signal-processing unit can comprise gate arrangements designed to select certain echoes in advance.

The signal-processing unit can also include a peak-value-detecting analog-digital convertor. This convertor can then detect the amplitude of the reflected ultrasonic signals (echoes) and these analog echo amplitude values are then converted to corresponding digital signals.

The signal-processing unit can also comprise or be connected to a computer. This computer can carry out the calculation and recording tasks in question such as, for example, production of ratios and differences, and also the amplitude mean values and the spread of these.

Finally, the signal-processing unit can comprise or be connected to an attenuator by means of which the amplitude of the received echoes can be adjusted to a suitable reference level.


## ADVANTAGES

The method and devices described above have made it possible to determine with great accuracy the degree of adherence between an explosive and a delimiting surface. In studies connected with the present invention it has been possible to detect as small a gap as 0.001 mm by means of the invention, something which has not been possible with previously known methods.

The amplitude of the ultrasonic signal reflected from the join between the explosive and the delimiting surface thus represents an extremely good measurement of the degree of adherence. The weaker this reflected signal is, the better the adherence, and vice versa. Little reflection means that the signal is penetrating further into the explosive lying behind.

By supplementing the measurement series using reflections from the join between the explosive and delimiting wall (inner surface) with a series, during the same measurement run, carried out using reflections from the outer surface of the delimiting wall and by showing the result as a ratio of the amplitude values from these two measurement series, the assessment is facilitated and, at the same time, correction for variations in the mains voltage is obtained.

The shape and structure of the delimiting wall and also the thickness of the lacquer layer which may have been applied for increasing the adherence can exhibit considerable variations. As regards the question of the thickness of the lacquer layer, this can vary especially at the base of a shell, which is a position where, as has been mentioned above, control of the adherence is of particularly great interest. In order to be able to make corrections for these variations, before the measurement series with applied explosive (main measurement run) takes place, corresponding measurement series are carried out with respect to the delimiting wall without applied explosive (preparatory measurement run). In this latter measurement run, however, any lacquer layer used will have been applied. Calculating the difference between the measurement series ratios from the two measurement runs provides a value in which account has been taken of both the structure of the delimiting wall and of any variations in the thickness of the lacquer layer. The reflection from a lacquered surface is generally greater than that from a non-lacquered surface, which can be explained by the fact that small irregularities have been filled with the lacquer.

Having the abovementioned signal-processing unit also comprise one or more gate arrangements affords the possibility of selecting certain echoes in advance.

The signal-processing unit can also comprise a peak-value-detecting analog-digital convertor and, by means of the latter, the amplitudes of the received reflected ultrasonic signals are converted to corresponding digital signals. These digital signals can be processed advantageously in a computer which, in addition to the desired ratios and differences, can also calculate the mean values and standard deviations from the mean value of the amplitudes. In this way a certain amount of information can be obtained regarding the regularity of the measured signals of the adherence.

An attenuator can also be included in or can be connected to the signal-processing unit. By means of this attenuator, the echo picked up can be adjusted to a suitable, fixed reference level. At excessively low levels the back noise can have too great an effect, while excessively high levels can cause non-linearities.

## DESCRIPTION OF FIGURES

An embodiment of the present invention is described below with reference to the attached drawings, in which

Figure 1 shows schematically, and in the form of a block diagram, how the invention can be applied in the case of the base part of a shell filled with explosive, and

Figure 2 again illustrates schematically how the invention can be applied in the case of a shell casing which will be filled later with an explosive.

## DETAILED EMBODIMENT

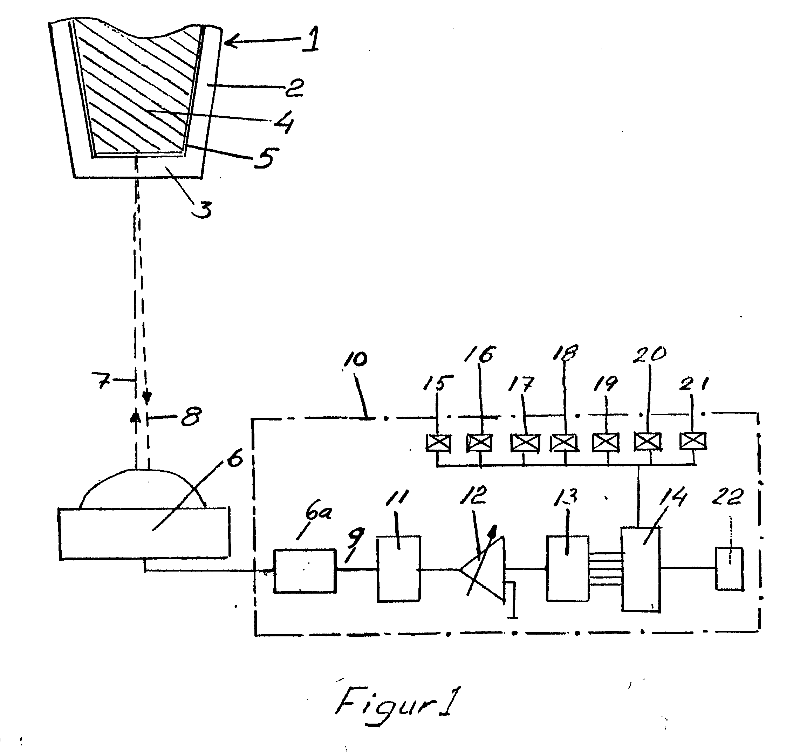

The principle of the present invention is shown in Figure 1. This shows the rear part of a shell 1 which is to be tested in accordance with the invention. The figure shows the rear part of the shell casing 2 and the base plate 3 of the shell. The shell is filled with an explosive 4, and lacquer layer 5 is applied between this explosive and the shell casing and base plate. The purpose of this lacquer layer is to improve the adherence between the explosive and the shell casing.

An ultrasonic source 6 is arranged at a distance from the base plate 3 of the shell. This source will be able to send ultrasonic signals in the frequency range 100 Khz -50 Mhz and also receive reflected ultrasonic signals (echoes). In the case illustrated in Figure 1, the ultrasonic source is directed towards the join between the explosive 4 and the surface of the base plate 3 facing the explosive (= inner surface). Ultrasonic signals 7 emitted from the ultrasonic source 6 and received reflected signals (echoes) 8 are in principle parallel and take place separately in time. However, for reasons of clarity, they have been shown in Figure 1 at a small relative angle.

The ultrasonic crystal 6 and the ultrasonic apparatus 6a (transmitter and receiver) are in communication via the line 9 with the signal-processing unit 10, which receives predetermined echoes at certain selected intervals. The selection of these echoes from the ultrasonic apparatus 6a is dealt with by the gate arrangement 11. Following this is the adjustable attenuator 12 where received echoes are adjusted to a predetermined reference level, inter alia for the purpose of preventing errors due to non-linearity of the equipment. The output of the attenuator 12 is connected to a peak-value-detecting analog-digital convertor 13 in which the amplitude of the received echo adjusted in the attenuator 12 is converted from analog character to binary figures. In this way the received echo can be recorded and processed in the computer 14.

The reflected ultrasonic signal which is illustrated in Figure 1 represents an echo from the inner surface facing the explosive during measurement of an explosive-filled shell (main measurement run). This type of echo has been designated as the main-inner surface-echo amplitude value ( = MIE), and the mean value of this echo type is calculated by the computer 14 and can be stored in a storage or memory space which is symbolized in Figure 1 by reference 15. In the same measurement run with a filled shell ( = main measurement run), the mean values of echoes from the outer surface of the base plate 3 are also calculated. These latter values are designated as the main - outer surface - echo amplitude values (MOE) and correspond to the memory space 16. The computer 14 also calculates the MIE/MOE ratio which is represented by the memory space 17.

As has been mentioned in the earlier description, the main measurement run (with filled shell) is to be preceded by a preparatory measurement run (with empty shell), and the mean values PIE and POE obtained from these measurement series and also the PIE/POE ratio are recorded and calculated by the computer 14. The memory spaces 18, 19 and 20 are used in this case. Finally, the difference between the two ratios is also calculated and is stored in the memory space 21. The different values in the memory spaces 15-21 can be obtained separately or together on the print-out member 22 connected to the computer 14.

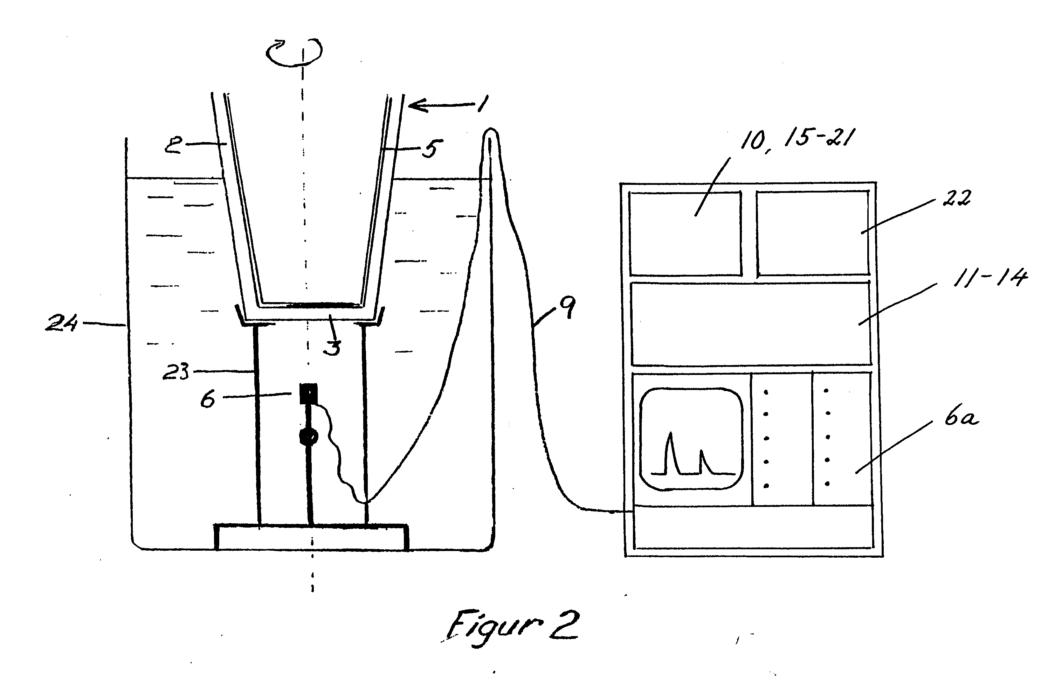

The invention has been used, inter alia, on a 15.5 cm series of m/77 high-explosive shells, and this application will now be described in further detail with reference to Figure 2.

The empty shell casing (2 + 3), which is made of steel and weighs approximately 35 kg, is placed in a rotatable gig 23 which is immersed in a water-filled container 24. The water serves as the contact agent between the ultrasonic source 6 and the outer surface of the base plate 3 of the shell. Both the casing 2 of the shell and its base plate 3 are provided with a lacquer layer 5 intended to increase the adherence between the shell casing (2 + 3) and the explosive 4 with which the shell is to be subsequently filled. The ultrasonic source 6 (USE 1 model from Krautkrämer) emits ultrasonic signals of a frequency of about I Mhz, and the direction is adjusted until the maximum amplitude is obtained for the echo from the outer surface of the base plate 3 of the shell. In order to reduce the influence of small irregularities in the shell surface, the shell 1 is rotated gently during the measurement. Each measurement series includes 50 consecutive echoes from the same surface. The signals generated by the ultrasonic source 6 pass through the line 9 to the signal-processing unit 10. In order for the correct echo to be selected from among the analog signals emitted from the ultrasonic source 6, a marking pulse is generated at 11. This pulse is adjustable in time and is shown on the screen of the ultrasonic apparatus. The marking pulse is placed above the echo which is to be differentiated and is also accessible to the gate arrangement 11 which is included in the signal-processing unit 10. So that the attenuator 12 (model 5893 from Optical Electronics) also included in this unit does not load the ultrasonic apparatus and thereby cause measurement errors, the output unit thereof has been supplemented with a circuit (type 2535 from Harris). The attenuator 12 is set at a predetermined reference level in such a way that the best possible amplification and band width over a large frequency range are obtained.

The signal-processing unit also includes the peak-value-detecting analog-digital converter 13 (ZD 460 from Zeltex) which shows the output signal in BCD form since the interface (11203A from Hewlett Packard) of the connected computer 14 (9820A from Hewlett Packard) is designed for this code.

In a measurement series of 50 consecutive amplitudes, the amplitudes of the echoes from the same surface are recorded by the computer 14, which also calculates the mean value and the mean error of the mean value. These values and the attenuation setting of the attenuator 12 and the number of the shell are memorized by the computer.

After this measurement series, the marking pulse is moved so that it covers the echo from the inner surface of the base plate 3 of the shell. The attenuation is then adjusted so that the amplitude of the echo now in question is approximately the same size as in the preceding measurement series. This is carried out in order to avoid errors due to non-linearity in the equipment. The computer 14 records 50 new amplitude values from the inner surface of the base plate 3 and calculates the mean value and standard deviation and also memorizes these together with the new attenuation setting and the number of the shell. The ratio between the inner surface mean values and the outer surface mean values is also calculated.

The measurement series described above relate to what was designated earlier as the preparatory measurement run, and the amplitudes are called

preparatory - outer surface - echo amplitude (POE) and

preparatory - inner surface - echo amplitude (PIE).

When these two measurement series have been carried out, the shell is filled with explosive, in this case with approximately 8 kg of trinitrotoluene. The filled shell is then subjected ( = main measurement runs to corresponding measurements which give

main - outer surface - echo amplitude (MOE) and

main - inner surface - echo amplitude (MIE)

and the MIE/MOE ratio.

As a very good measurement of adherence, the computer finally calculates the difference PIE/POE - MIE/MOE.

The measurements described above with reference to 15.5 cm m/77 high-explosive shells included 10 different shells. In addition to the measurement of the outer surface echoes and inner surface echoes in empty shell casings and in casings after they had been filled in a customary manner with explosive, a measurement series was also carried out after the shells had been subjected to an additional treatment (temperature shock) which, experience shows, provides increased adherence between the explosive and the shell casing. The values obtained are to be found in the attached table (see page 13) and agree with what was calculated theoretically and was found in model tests. Thus, the shells exhibit a greater difference between the ratios in question after the temperature shock. The spread shown as standard deviation varied between 0.1 and 0.2.

The invention has also been used on a series of 12-cm naval target shells, m/70, in which the total weight of the shell was 24.6 kg, of which 2.6 kg consisted of trinitrotoluene. The filling with explosive was carried out under conditions which varied slightly. Most of the shells exhibited satisfactory adherence, with ratio differences of between 6.1 and 15.1, while in a small number of shells the difference was only between 0 and 3.5 and the adherence was therefore less good.

The invention is not limited to the embodiment shown above by way of example, but instead can be modified within the scope of the following patent claims and the inventive concept.

| Shell no. | Empty shell casing $\frac{PIE}{POE}$ | Shell with explosive $\frac{MIE}{MOE}$ | Shell with explosive $\frac{PIE}{POE} - \frac{MIE}{MOE}$ | Shell after temperature shock $\frac{MIE}{MOE}$ | Shell after temperature shock $\frac{PIE}{POE} - \frac{MIE}{MOE}$ |
|---|---|---|---|---|---|
| 1 | 6.3 | 6.1 | 0.2 | 5.2 | 1.0 |
| 2 | 6.0 | 4.2 | 1.8 | 3.7 | 2.2 |
| 3 | 6.1 | 5.5 | 0.6 | 5.0 | 1.1 |
| 4 | 6.3 | 4.7 | 1.6 | 4.7 | 1.6 |
| 5 | 6.1 | 6.0 | 0.2 | 4.5 | 1.6 |
| 6 | 6.2 | 4.9 | 1.2 | 4.5 | 1.7 |
| 7 | 6.1 | 5.7 | 0.4 | 4.9 | 1.2 |
| 8 | 6.1 | 4.9 | 1.2 | 4.7 | 1.4 |
| 9 | 6.1 | 5.4 | 0.7 | 4.7 | 1.4 |
| 10 | 6.0 | 4.8 | 1.2 | 4.5 | 1.4 |

**Claims**

1. Method for determining the degree of adherence between an explosive (4) and the delimiting surface in a delimiting wall (2, 3) of a projectile for example the base part (3) of a shell, characterized in that
   (a) ultrasonic signals are emitted, from an ultrasonic source (6) situated outside the delimiting wall, in the direction of the join between the explosive and the delimiting wall, and reflected ultrasonic signals (echoes) are received and recorded,
   (b) in a preparatory measurement run in which the explosive has not yet been applied to the inside of the delimiting wall, but only, if appropriate, a lacquer layer (5) which is designed to improve the adherence of the explosive against the delimiting surface, the emitted ultrasonic signals consist of two different series directed towards
   on the one hand, the inner surface of the delimiting wall and
   on the other hand, the outer surface of the delimiting wall,
   and the amplitude values for the corresponding echoes are recorded, i.e.

7

preparatory - inner surface - echo amplitude value (PIE) and

preparatory - outer surface - echo amplitude value (POE)

      c) in a main measurement run in which the explosive (4) has been applied against the delimiting surface, the emitted ultrasonic signals consist in the same way of two different series directed towards on the one hand, the inner surface of the delimiting wall and

on the other hand, the outer surface of the delimiting wall,

and the amplitude values for the corresponding echoes are recorded, i.e.

main - inner surface - echo amplitude value (MIE) and

main - outer surface - echo amplitude value (MOE),

      (d) and the ratios PIE/POE and MIE/MOE and the difference between these two ratios are calculated, this difference representing a very good measurement of the degree of adherence between the explosive and the delimiting surface, in which respect a greater difference indicates better adherence.

      2. Device for carrying out the method stated in Claim 1 for determining the degree of adherence between an explosive (4) and a delimiting surface and the delimiting wall (2, 3) of a projectile, characterized in that an ultrasonic source (6) is arranged outside the delimiting wall for emitting series of ultrasonic signals on the one hand towards the inner surface of the delimiting wall and on the other hand to its outer surface both in a preparatory measurement run and in a main measurement run, in that equipment is provided for receiving reflected ultrasonic signals (echoes), which receiving can be carried out either by the ultrasonic source or another unit, and in that a signal-processing unit (10) is designed to record the echoes obtained.

      3. Device according to Patent Claim 2, characterized in that the signal-processing unit comprises gate arrangements (11) designed to select certain echoes in advance.

      4. Device according to Patent Claim 2 or 3, characterized in that the signal-processing unit comprises a peak-value-detecting analog-digital convertor (13) which detects the amplitude of reflected ultrasonic signals (echoes) and converts these to corresponding digital signals.

      5. Device according to any one of Patent Claims 2-4, characterized in that the signal-processing unit comprises or is connected to a computer (14) which carries out the calculating and recording tasks in question such as, for example, calculating the ratios and differences and also the amplitude mean values and the spread of these.

      6. Device according to any one of Patent Claims 2-5, characterized in that the signal-processing unit comprises or is connected to an attenuator (12) which can adjust the amplitude of the received echoes to a suitable reference level.

Figur1

Figur 2

EP 0 381 653 A2